(19) Europäisches Patentamt
European Patent Office
Office européen des brevets

(11) EP 4 545 582 A1

(12) **EUROPEAN PATENT APPLICATION**
published in accordance with Art. 153(4) EPC

(43) Date of publication:
**30.04.2025 Bulletin 2025/18**

(21) Application number: **22965508.9**

(22) Date of filing: **17.11.2022**

(51) International Patent Classification (IPC):
***C08G 18/71*** (2006.01) ***C08G 18/75*** (2006.01)
***C08G 18/76*** (2006.01) ***C08G 18/77*** (2006.01)
***C08G 18/78*** (2006.01) ***C08G 18/79*** (2006.01)
***C08G 18/66*** (2006.01) ***C08G 18/42*** (2006.01)
***C08G 18/32*** (2006.01) ***C07C 265/14*** (2006.01)

(52) Cooperative Patent Classification (CPC):
(C-Sets available)
**C07C 265/14; C07C 263/20; C08G 18/10; C08G 18/244; C08G 18/3206; C08G 18/4236; C08G 18/724; C08G 18/751; C08G 18/7614; C08G 18/7678** (Cont.)

(86) International application number:
**PCT/CN2022/132424**

(87) International publication number:
**WO 2024/103324 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Wanhua Chemical Group Co., Ltd.**
**Yantai, Shandong 264006 (CN)**

(72) Inventors:
- **ZHU, Fulin**
  **Yantai, Shandong 264006 (CN)**
- **SHANG, Yonghua**
  **Yantai, Shandong 264006 (CN)**
- **LI, Jianfeng**
  **Yantai, Shandong 264006 (CN)**
- **HE, Wei**
  **Yantai, Shandong 264006 (CN)**
- **WU, Qian**
  **Yantai, Shandong 264006 (CN)**
- **WANG, Qinlong**
  **Yantai, Shandong 264006 (CN)**
- **JIANG, Tengfei**
  **Yantai, Shandong 264006 (CN)**
- **LI, Qiang**
  **Yantai, Shandong 264006 (CN)**
- **JIA, Zhengrui**
  **Yantai, Shandong 264006 (CN)**
- **LI, Yuan**
  **Yantai, Shandong 264006 (CN)**

(74) Representative: **Potter Clarkson**
**Chapel Quarter**
**Mount Street**
**Nottingham NG1 6HQ (GB)**

# (54) ISOCYANATE COMPOSITION, MODIFIED COMPOSITION AND POLYURETHANE ELASTOMER

(57) Disclosed in the present invention are an isocyanate composition, a modified composition, and a polyurethane elastomer, the isocyanate composition having an effective factor of 3.80-5.30. By means of designing and controlling the effective factor, the isocyanate composition has excellent reaction activity and thus can be used for preparing high-performance polyurethane products. The isocyanate composition can improve stability of polyurethane products, and particularly, can remarkably improve resistance to color changes and weather resistance of polyurethane elastomers, suppress increases of color codes and yellowing under humid and hot conditions, and improve the tensile strength and tearing strength of polyurethane elastomers, such that the polyurethane elastomers have excellent comprehensive performance in the areas such as weather resistance, stability and mechanical properties.

EP 4 545 582 A1

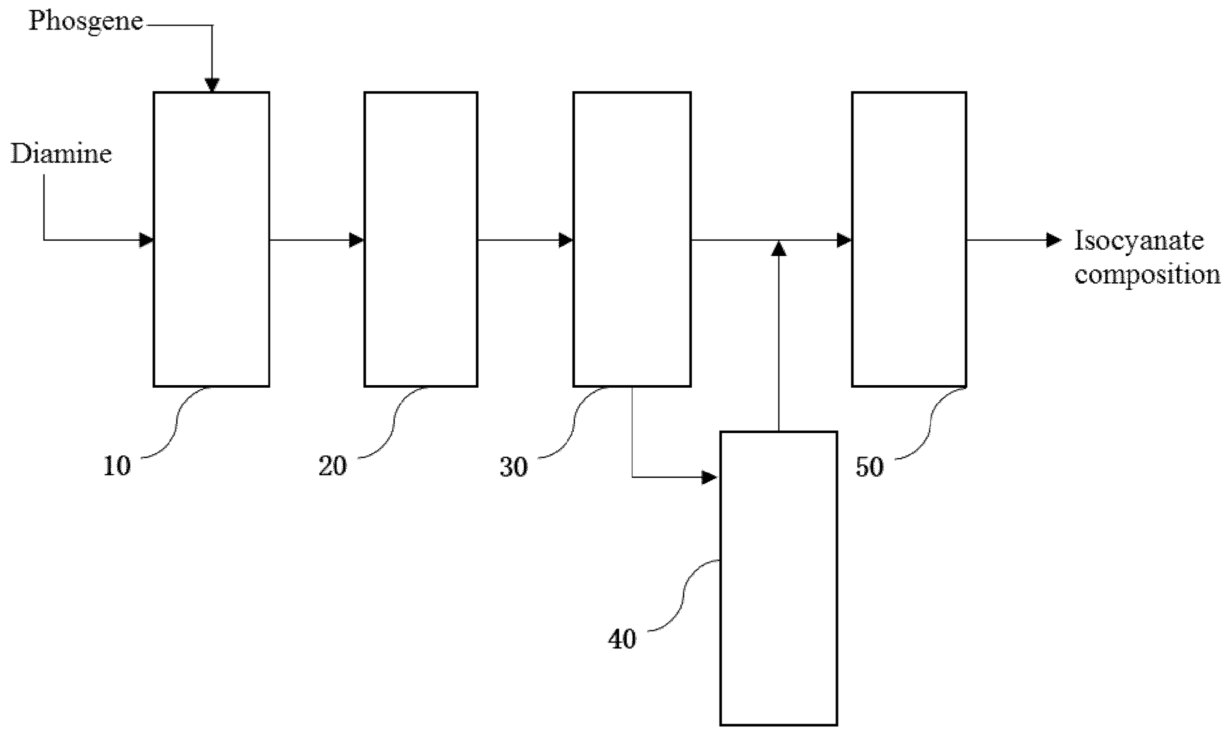


FIG. 1

(52) Cooperative Patent Classification (CPC): (Cont.)

C-Sets
**C07C 263/20, C07C 265/14;**
**C08G 18/10, C08G 18/3206**

## Description

### FIELD OF THE INVENTION

Embodiments of the present application relate to the technical field of isocyanates, such as an isocyanate composition, a modified composition and a polyurethane elastomer.

### BACKGROUND OF THE INVENTION

Polyurethane elastomers (PUR) are characterised by high elasticity, high strength, a wide range of hardness, and good wearability, and are used in the automotive industry, the machinery industry, the medical industry, transportation, sporting goods, the electronics industry, the chemical industry, coal industry and so on. Polyurethane elastomers can be classified into TDI (toluene diisocyanate) type, MDI (diphenylmethane diisocyanate) type, PPDI (paraphenylene diisocyanate) type, NDI (naphthalene diisocyanate) type, CHDI (cyclohexane diisocyanate) type and so on according to different isocyanates used, with TDI and MDI types being the most common.

With the expansion of polyurethane elastomer applications, the commonly used TDI-type PURs and MDI-type PURs can no longer satisfy the usage requirements. Therefore, new types of polyurethane elastomers have been developed in order to obtain an improvement in elastomer performance. For example, a polyurethane elastomer with good heat resistance and anti-sliding property is disclosed in CN113354788A, the raw material comprises 60%-65% of polyester polyol, 30%-33% of diisocyanate, 1%-9.5% of mixed chain extender and crosslinking agent, and the diisocyanate comprises a combination of dimethyl biphenyl diisocyanate, p-phenylene diisocyanate and 1,5-naphthalene diisocyanate. In CN104017166A, a method for preparing a high-temperature-resistant thermoplastic polyurethane elastomer is disclosed, the method comprises the following steps: adding an antioxidant, a catalyst and a molten polyester polyol to a reactor, and dehydrating under heating and reduced pressure to form component A; heating and melting a diisocyanate to form component B; mixing a molten ternary alcohol and a molten diol uniformly, and dehydrating under reduced pressure and heating to form component C; adding component A, component B and component C simultaneously to a twin-screw extruder, and obtaining the high-temperature-resistant thermoplastic polyurethane elastomer by a stepwise polymerization reaction; wherein the diisocyanate is a mixture consisting of 70%-100% by weight of trans-1,4-CHDI and 0-30% by weight of cis-1,4-CHDI. In CN104817683A, a polyurethane elastomer with good mechanical properties and fatigue resistance is disclosed, wherein the raw material comprises component A and component B. Component A comprises 100 parts by weight of a macromolecular diol, 5-20 parts by weight of NDI, 10-30 parts by weight of p-phenylene diisocyanate or 10-50 parts by weight of 3,3'-dimethyl-4,4'-biphenylene diisocyanate (TODI); and component B comprises 0-100 parts by weight of a macromolecular diol, 8-30 parts by weight of a chain extender, and 0.02-0.5 parts by weight of a catalyst. The weight ratio of component A to component B is 100:(8-30).

Compared with the commonly used MDI and TDI, NDI, PPDI and CHDI have higher melting point and belong to high melting point isocyanate. Moreover, NDI and PPDI possess aromatic structures and significant steric hindrance effect, thus the synthesized polyurethane elastomers have the characteristics such as high hardness, good resilience, good heat resistance, excellent dynamic performance, good wearability, etc., which can be applied to the high dynamic load scenarios, and is a favored isocyanate raw material for preparing new types of polyurethane elastomers. However, despite the various advantages of NDI, PPDI, and CHDI, there are also drawbacks that cannot be ignored. Due to the high reactivity of NCO groups, they may cause unnecessary color increase during the production of polyurethane, that is, obvious yellowing occurs, and weather resistance is poor, which seriously affects the appearance and the product quality of polyurethane elastomers.

Therefore, there is an urgent need in this field to develop isocyanate raw materials with better properties to achieve improved properties such as weather resistance and appearance of polyurethane elastomers.

### SUMMARY OF THE INVENTION

The following is a summary of the subject matter described in detail herein. This summary is not intended to limit the scope of protection of the claims.

Embodiments of the present application provide an isocyanate composition, a modified composition and a polyurethane elastomer. Through the design of an effective factor, the isocyanate composition can effectively inhibit the color increase of the polyurethane elastomer under humid and hot conditions, significantly improve the stability and weather resistance of the polyurethane elastomer, and improve the mechanical properties of the polyurethane elastomer.

In a first aspect, an embodiment of the present application provides an isocyanate composition, and an effective factor of the isocyanate composition is in a range from 3.80 to 5.30.

The calculation formula of the effective factor is shown in Formula I:

$$E = -\lg\left(A - \frac{B \times M_{Cl}}{M_B}\right) \quad \text{Formula I.}$$

In Formula I, E is the effective factor.

In Formula I, A is the mass content of chlorine in the isocyanate composition.

In Formula I, B is the mass content of chloroisocyanate in the isocyanate composition.

In Formula I, $M_{Cl}$ is the relative atomic mass of chlorine, which is about 35.5.

In Formula I, $M_B$ is the relative molecular mass of the chloroisocyanate.

The effective factor E of the isocyanate composition provided by the present application is in a range from 3.80 to 5.30, for example, it can be 3.90, 4.00, 4.10, 4.30, 4.50, 4.70, 4.90, 5.00, 5.10, or 5.20, as well as the specific point values between the above point values. Due to the limited space and for the sake of brevity, the present application does not provide an exhaustive list of specific point values included in the range.

In the present application, the isocyanate composition includes a combination of isocyanates and chlorine-containing substances, and is therefore named "isocyanate composition"; the chlorine-containing substance includes a combination of chloroisocyanates and substances corresponding to the effective factor. In the present application, by designing and controlling the effective factor, the isocyanate composition comprises a specific content and a specific type of chlorine-containing substance, so that it has excellent reactivity and can be used in the preparation of high-performance polyurethane products. In particular, the isocyanate composition, when used in polyurethane products, especially polyurethane elastomers, can significantly enhance the stability and weather resistance of the polyurethane elastomers, inhibit color increase and the yellowing under the humid and hot conditions, improve the tensile strength and the tear strength of the polyurethane elastomers, and enable the polyurethane elastomers to have an excellent overall performance in terms of weather resistance, stability, mechanical properties, and appearance, and the like.

Preferably, the isocyanate is diisocyanate, further preferably comprising any one or a combination of at least two of naphthalene diisocyanate (NDI), phenylene diisocyanate (PPDI/MPDI/OPDI), cyclohexane diisocyanate (CHDI), diphenylmethane diisocyanate (MDI), and toluene diisocyanate (TDI).

Unless otherwise specified, the isocyanates listed in the present application include all isomers thereof, for example, the naphthalene diisocyanate (NDI) is

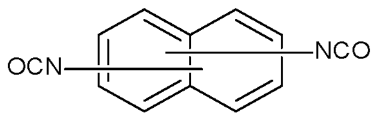

,

the phenylene diisocyanate (PPDI) is

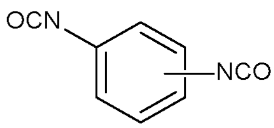

,

the cyclohexane diisocyanate (CHDI) is

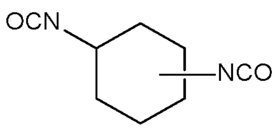

,

the diphenylmethane diisocyanate (MDI) is

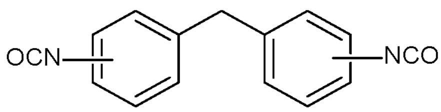

,

the toluene diisocyanate (TDI) is

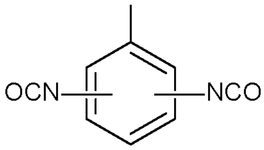, preferably 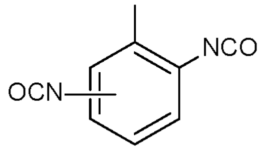.

Preferably, the naphthalene diisocyanate is 1,5-naphthalene diisocyanate (

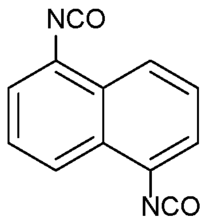

); the benzene diisocyanate is 1,4-benzene diisocyanate (p-phenylene diisocyanate,

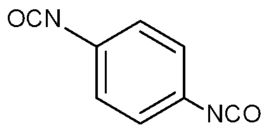

); the cyclohexane diisocyanate is 1,4-cyclohexane diisocyanate (

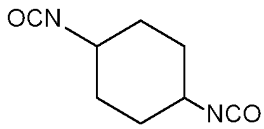

); the diphenylmethane diisocyanate is 4,4'-diphenylmethane diisocyanate (

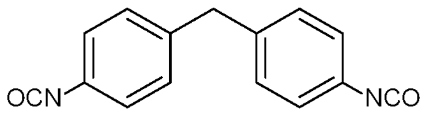

), the toluene diisocyanate is 2,4-toluene diisocyanate (

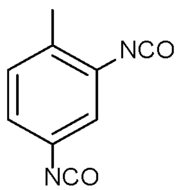

) and/or 2,6-toluene diisocyanate (

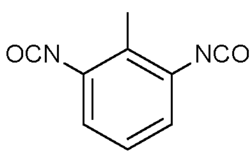

).

Preferably, the mass percentage of isocyanate in the isocyanate composition is ≥97%, for example, it can be 97.5%, 98%, 98.5%, 99%, 99.1%, 99.2%, 99.3%, 99.4%, 99.5%, 99.6%, 99.7%, 99.8%, 99.9%, 99.92%, 99.95%, 99.98%, 99.99%, etc., further preferably ≥99%, more preferably >99%.

Preferably, the chloroisocyanate is a compound obtained by replacing one NCO group in isocyanate with chlorine.

Preferably, the chloroisocyanate comprises any one or a combination of at least two of

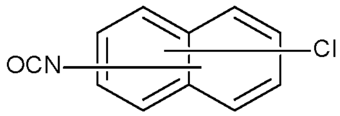 (preferably 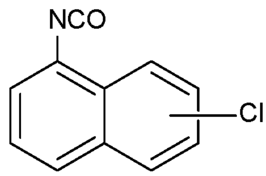,

chloronaphthalene isocyanate CNI, the isocyanate being NDI),

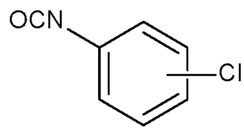

(chlorophenyl isocyanate CPPI, the isocyanate being PPDI),

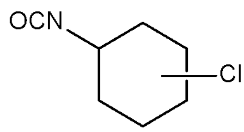

(chlorocyclohexyl isocyanate CCHI, the isocyanate being CHDI),

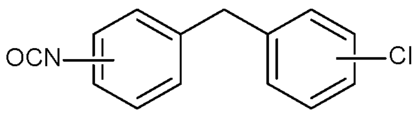

(preferably

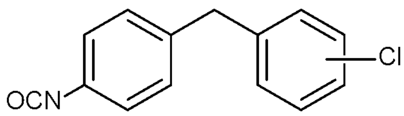,

chlorodiphenylmethane isocyanate, the isocyanate being MDI),

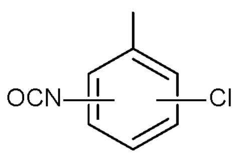

(preferably

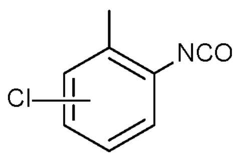,

chlorotoluene isocyanate, the isocyanate being TDI).

In the present application, an expression for a ring structure having a '-' indicates that the attachment site is at any position on the ring structure that is capable of bonding.

Preferably, the mass content (B-value) of the chloroisocyanate in the isocyanate composition is in a range from 5 ppm to 2000 ppm, for example, it can be 10 ppm, 20 ppm, 30 ppm, 50 ppm, 80 ppm, 100 ppm, 300 ppm, 500 ppm, 700 ppm, 900 ppm, 1000 ppm, 1100 ppm, 1300 ppm, 1500 ppm, 1700 ppm, or 1900 ppm, as well as specific point values between the above point values. Due to the limited space and for the sake of brevity, the present application does not provide an exhaustive list of specific point values included in the range, and a range from 10 ppm to 1500 ppm is further preferred.
In the present application, "ppm" means parts per million, and 1 ppm means parts per million; when the same expressions are used hereinafter, they shall have the same meaning.
Preferably, the substance corresponding to the effective factor comprises any one or a combination of at least two of the following compounds:

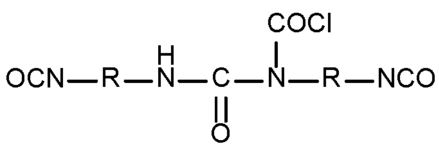 , 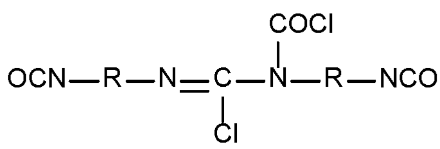 ,

and

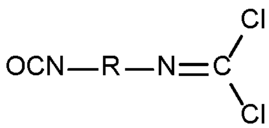 ,

wherein R is a divalent group obtained by removing NCO groups in isocyanate.
Preferably, R is selected from any one or a combination of at least two of

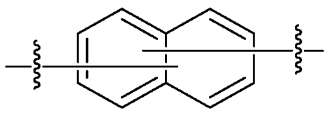

(the isocyanate being NDI),

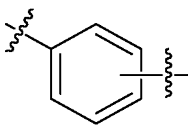

(the isocyanate being (the isocyanate being NDI), (the isocyanate being PPDI),

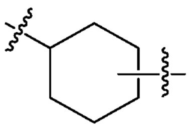

(the isocyanate being CHDI),

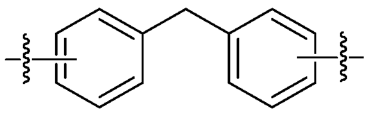

(the isocyanate being MDI) and

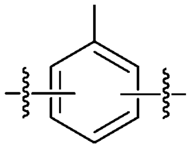

(preferably, the isocyanate being TDI); wherein a wavy line represents the connection sites of groups.

Preferably, the mass content (A-value) of chlorine in the isocyanate composition is in a range from 1 ppm to 1000 ppm, for example, it can be 2 ppm, 5 ppm, 8 ppm, 10 ppm, 30 ppm, 50 ppm, 80 ppm, 100 ppm, 150 ppm, 200 ppm, 250 ppm, 300 ppm, 350 ppm, 400 ppm, 450 ppm, 500 ppm, 550 ppm, 600 ppm, 650 ppm, 700 ppm, 750 ppm, 800 ppm, 850 ppm, 900 ppm, or 950 ppm, as well as specific point values between the above point values. Due to the limited space and for the sake of brevity, the present application does not provide an exhaustive list of specific point values included in the range, and a range from 5 ppm to 500 ppm is further preferred.

In Formula I for calculating the effective factor of the present application, A is the mass content of chlorine in the isocyanate composition, which is preferably obtained by X-ray fluorescence spectrometry (XRF).

Preferably, in Formula I for calculating the effective factor, B is the mass content of chloroisocyanate in the isocyanate composition, which is obtained by chromatography-mass spectrometry, preferably by gas chromatography-mass spectrometry (GCMS).

According to the research of the present application, it was found that the characterization methods for chlorine content in isocyanate known in the related art are difficult to accurately control the performance of isocyanates, and thus the performance of polyurethane products/polyurethane elastomers, especially yellowing resistance, stability and appearance, cannot be effectively controlled. Specifically, in standard GB/T 12009.1-1989, the method for testing total chlorine content is the oxygen bottle combustion method, which converts all chlorine (including bromine) in isocyanates into inorganic chlorine (including bromine) and then titrates with silver nitrate to characterize all chlorine content in isocyanate, including the content of bromine in isocyanate. In standard GB/T 12009.2-2016, the content of hydrolysable chlorine is determined, specifically the chlorine released after the reaction of isocyanate with alcohol and water, which is the chlorine with higher activity in isocyanate, including bromine with higher activity, and some monochloroisocyanates can also hydrolyze a part. The content of chlorine (including a part of bromine) determined by GB/T 12009.1-1989 or GB/T 12009.2-2016 cannot accurately represent the composition information of isocyanates, therefore cannot effectively control the performance of isocyanates, polyurethane products, especially polyurethane elastomers.

As a preferred technical solution of the present application, in the calculation of the effective factor E, A is the total chlorine content (excluding bromine) obtained by XRF testing, and B is the chloroisocyanate content obtained by chromatography-mass spectrometry, and the A value and the B value are obtained by testing in a precise qualitative and quantitative analytical method, so as to enable the effective factor E to accurately characterise the polychlorinated substituents as well as a portion of the hydrolysable chlorine in the isocyanate compositions (excluding the hydrolysable chlorine of monochloroisocyanates) in the isocyanate compositions, corresponding to a finer and more defined chlorine content, which plays a key role in the activity of the isocyanate as well as in the performance of the polyurethane products (such as polyurethane elastomers), thereby achieving the performance regulation of the isocyanate composition, and improving the performance of the polyurethane elastomers prepared therefrom, especially with significant enhancement effects on the weather resistance, stability, mechanical properties and appearance.

It should be noted that in the present application, the chloroisocyanate and the substance corresponding to the effective factor can be produced as by-products in the preparation of isocyanates, or artificially added to obtain the required content.

In a second aspect, embodiments of the present application provide a method for preparing the isocyanate composition as described in the first aspect, and the method comprises: reacting an amine compound with a phosgene to obtain the isocyanate composition.

Preferably, the method comprises the following steps:

(1) reacting the amine compound with phosgene to obtain a reaction product;

(2) carrying out a removal treatment on the reaction product obtained in step (1) to obtain a crude product; wherein the removal treatment comprises a phosgene removal treatment and/or a solvent removal treatment; and

(3) separating and refining the crude product obtained in step (2) in turn to obtain the isocyanate composition.

It should be noted that when diamine and phosgene are used for isocyanate-formation reaction, specific effective factors can be obtained by optimizing the following parameters. The effective factor E of the isocyanate composition can also be adjusted by adding chloroisocyanate and/or a substance corresponding to the effective factor to the isocyanate.

Preferably, a heavy component and an intermediate are obtained by the separating in step (3); and a mixture of the intermediate and the heavy component is refined to obtain the isocyanate composition; and the mass percentage of the heavy component in the mixture is in a range from 1% to 10%.

As a preferred technical solution of the present application, the component to be refined is a mixture of the intermediate and the heavy component, and the mass percentage of the heavy component in the mixture is in a range from 1% to 10%, for example, it can be 2%, 3%, 4%, 5%, 6%, 7%, 8% or 9%, as well as specific point values between the above point values. Due to the limited space and for the sake of brevity, the present application does not provide an exhaustive list of specific point values included in the range, and a range from 2% to 10% is further preferred.

Preferably, the heavy component obtained by the separation may be directly mixed with the intermediate to obtain a mixture; or, the heavy component obtained by separation is a primary heavy component, the primary heavy component is separated again to obtain a heavy component recovery material and a residual heavy component; the heavy component recovery material is mixed with the intermediate to obtain the mixture; and the mass percentage content of the heavy component recovery material in the mixture is in a range from 1% to 10%.

In another preferred technical solution, the method for preparing the isocyanate composition comprises: mixing the isocyanate obtained by carbamate cracking with the heavy component recovery material to obtain the isocyanate composition. Preferably, the mass percentage of the heavy component recovery material in the isocyanate composition is in a range from 1% to 10% (for example 2%, 3%, 4%, 5%, 6%, 7%, 8% or 9%), further preferably in a range from 1% to 5%.

As a preferred technical solution of the present application, the method for preparing the isocyanate composition is the phosgenation method, that is, amine compounds are reacted with phosgene to produce isocyanate; the amine compounds include diamine and/or diamine salts (for example, diamine hydrochloride obtained by reacting diamine with HCl).

Preferably, the methods of reacting amine compounds with phosgene exemplarily comprise the following three categories: methods of reacting diamine with phosgene in gas phase, also known as gas phase phosgenation method; methods of reacting a diamine with phosgene in the liquid phase, also known as liquid phase phosgenation or cold and hot two-stage phosgenation; and methods of reacting a diamine salt (for example, diamine hydrochloride) with phosgene in a solvent, also known as phosgenation of diamine hydrochloride; wherein cold and hot two-stage phosgenation is further preferred in the present application.

Preferably, the amine compound in step (1) is a diamine, and the reaction in step (1) is the cold and hot two-stage phosgenation.

Preferably, step (1) specifically comprises: mixing the diamine with a solvent to obtain an amine solution; introducing a phosgene into the amine solution to carry out the reaction to obtain a reaction product, that is, a reaction solution containing diisocyanate.

Preferably, the solvent is an organic solvent, exemplarily including but not limited to: aromatic hydrocarbons such as benzene, toluene, xylene, etc.; aliphatic hydrocarbons such as octane, decane, etc.; alicyclic hydrocarbons such as cyclohexane, methylcyclohexane, ethylcyclohexane, etc.; halogenated aromatic hydrocarbons such as chlorotoluene, chlorobenzene, dichlorobenzene, dibromobenzene, trichlorobenzene, etc.; nitrogen-containing compounds such as nitrobenzene, N,N-dimethylacetamide, N,N'-dimethylimidazolidinone, etc.; ethers such as dibutyl ether, ethylene glycol dimethyl ether, ethylene glycol diethyl ether, etc.; ketones such as heptanone, diisobutyl ketone, methyl isobutyl ketone, methyl ethyl ketone, etc.; fatty acid esters such as ethyl acetate, butyl acetate, amyl acetate, ethoxy ethyl acetate, etc.; aromatic carboxylic acid esters such as methyl salicylate, dimethyl phthalate, dibutyl phthalate, methyl benzoate, etc.; the solvent can be used alone or in combination of at least two.

Preferably, the solvent comprises halogenated aromatic hydrocarbons, further preferably chlorobenzene and/or dichlorobenzene.

Preferably, the mass percentage of the diamine in the amine solution is in a range from 1 wt% to 50 wt%, for example, it can be 2 wt%, 5 wt%, 8 wt%, 10 wt%, 12 wt%, 15 wt%, 18 wt%, 20 wt%, 22 wt%, 25 wt%, 28 wt%, 30 wt%, 35 wt%, 40 wt%, 45 wt% or 48 wt%, as well as specific point values between the above point values. Due to the limited space and for the sake of brevity, the present application does not provide an exhaustive list of specific point values included in the range, and a range from 5 wt% to 40 wt% is further preferred.

Preferably, the molar ratio of the phosgene to the amine compound (diamine) in step (1) is in a range from 3:1 to 50:1, for example, it can be 4:1, 5:1, 8:1, 10:1, 12:1, 15:1, 18:1, 20:1, 22:1, 25:1, 28:1, 30:1, 32:1, 35:1, 38:1, 40:1, 42:1, 45:1 or 48:1, further preferably in a range from 4:1 to 40:1, and even further preferably in a range from 4:1 to 30:1.

Preferably, the reaction of step (1) comprises a cold reaction and a hot reaction carried out in sequence.

Preferably, the cold reaction is performed at a temperature in a range from -10°C to 80°C, for example, it can be -5°C, 0°C, 5°C, 10°C, 15°C, 20°C, 25°C, 30°C, 35°C, 40°C, 45°C, 50°C, 55°C, 60°C, 65°C, 70°C, or 75°C, as well as specific point values between the above point values. Due to the limited space and for the sake of brevity, the present application does not provide an exhaustive list of specific point values included in the range, and a range from 0°C to 70°C is further preferred.

Preferably, the cold reaction is performed for a time period of 1h to 20h, for example, it can be 2h, 3h, 4h, 5h, 6h,

7h, 8h, 9h, 10h, 11h, 12h, 13h, 14h, 15h, 16h, 17h, 18h or 19h, as well as specific point values between the above point values. Due to the limited space and for the sake of brevity, the present application does not provide an exhaustive list of specific point values included in the range, and a time period of 2h to 15h is further preferred.

Preferably, the hot reaction is performed at a temperature in a range from 70°C ro150°C, for example, it can be 75°C, 80°C, 85°C, 90°C, 95°C, 100°C, 105°C, 110°C, 115°C, 120°C, 125°C, 130°C, 135°C, 140°C, or 145°C, as well as specific point values between the above point values. Due to the limited space and for the sake of brevity, the present application does not provide an exhaustive list of specific point values included in the range, and a range from 80°C to 130°C is further preferred.

Preferably, the hot reaction is performed for a time period of 1h to 20h, for example, it can be 2h, 3h, 4h, 5h, 6h, 7h, 8h, 9h, 10h, 11h, 12h, 13h, 14h, 15h, 16h, 17h, 18h, or 19h, as well as specific point values between the above point values. Due to the limited space and for the sake of brevity, the present application does not provide an exhaustive list of specific point values included in the range, and a time period of 2h to 15h is further preferred.

Preferably, the reaction of step (1) is carried out under normal pressure or pressurized conditions.

Preferably, the pressure (gauge pressure) of the reaction in step (1) is in a range from 0 MPa G to 0.6 MPa G, for example, it can be 0.0005 MPa G, 0.001 MPa G, 0.003 MPa G, 0.01 MPa G, 0.02 MPa G, 0.03 MPa G, 0.05 MPa G, 0.07 MPa G, 0.09 MPa G, 0.1 MPa G, 0.2 MPa G, 0.3 MPa G, 0.4 MPa G, or 0.5 MPa G, as well as specific point values between the above point values. Due to the limited space and for the sake of brevity, the present application does not provide an exhaustive list of specific point values included in the range, a range from 0.005 MPa G to 0.4 MPa G is more preferred, and a range from 0.01 MPa G to 0.2 MPa G is even more preferred.

Preferably, the reaction (isocyanate-formation process) of step (1) is an intermittent process or a continuous process, preferably a continuous process.

The continuous process means that a slurry (amine solution) in a agitation tank is continuously transported from the agitation tank to a reaction tank different from the agitation tank, where the diamine is reacted with phosgene, and, the obtained reaction product (reaction solution containing diisocyanate) is continuously removed from the reaction tank. The present application does not specifically limit the number of reaction tanks for the continuous process, and exemplarily, it can be 2, 3, 4, 5 or more.

As needed, a removal treatment process (a solvent removal treatment process and/or a phosgene removal treatment process) and a separation and refining process may be carried out on the reaction product obtained in step (1).

Preferably, the phosgene removal treatment in step (2) is carried out in a tower for phosgene removal.

Preferably, the solvent removal treatment in step (2) is carried out in a tower for solvent removal.

Preferably, the separation in step (3) separates the intermediates (light components) from the heavy components to achieve the removal of the heavy components; exemplary devices for the separation include, but are not limited to: a short path evaporator, a rectification column, further preferably a short path evaporator.

Preferably, the short path evaporator has an operating pressure in a range from 0.05 kPa to 4 kPa, for example it can be 0.08 kPa, 0.1 kPa, 0.3 kPa, 0.5 kPa, 0.8 kPa, 1 kPa, 1.2 kPa, 1.5 kPa, 1.8 kPa, 2 kPa, 2.2 kPa, 2.5 kPa, 2.8 kPa, 3 kPa, 3.2 kPa, 3.5 kPa or 3.8 kPa, as well as specific point values between the above point values. Due to the limited space and for the sake of brevity, the present application does not provide an exhaustive list of specific point values included in the range, a range from 0.1 kPa to 2.5 kPa is further preferred.

As a preferred technical solution of the present application, the heavy components obtained from separation contain a richer variety and higher content of chlorine-containing substances. By incorporating the heavy components obtained from separation or the heavy component recovery material obtained from the secondary separation of the heavy components into the intermediates (light components) obtained by separation at a certain ratio, and then refining the same, the types and contents of chlorine-containing substances in the product can be effectively controlled, making the effective factor of the isocyanate composition ranging from 3.80 to 5.30.

Preferably, the mass percentage content of heavy components (heavy component recovery material) in the mixture (material involved in refining) is in a range from 1% to 10%, further preferably in a range from 2% to 10%, so that the effective factor of the isocyanate composition is in a range from 3.80 to 5.30. If the amount of heavy components (heavy component recovery material) is too small, the effective factor will be too high. When the isocyanate composition is used to prepare polyurethane elastomers, the reaction rate is too fast, resulting in uneven polymerization, which makes the mechanical properties of polyurethane elastomer worse and reduces the tensile strength and tear strength. If the amount of heavy components (heavy component recovery material) is too high, resulting in a low effective factor, the isocyanate composition contains more impurities, which makes the weather resistance of polyurethane elastomer worse, especially with obvious yellowing under humid and hot conditions.

Preferably, the heavy components mixed with the intermediates can be directly blended back into the intermediates, or can be recycled and separated by a heavy component removal device to obtain a heavy component recovery material, and then blended into the intermediates.

Preferably, the method for refining is an industrial separation technique known in the art, and exemplarily includes, but is not limited to: rectification, rectification, crystallization and the like.

Preferably, the method for refining is rectification.

Preferably, the rectification is carried out in a rectification column, and the rectification column preferably comprises a plate rectification column or a filled rectification column.

Preferably, the rectification column has a number of theoretical plates ranging from 2 to 40, for example, it can be 3, 5, 8, 10, 12, 15, 18, 20, 22, 25, 28, 30, 32, 35 or 38, as well as specific point values between the above point values. Due to the limited space and for the sake of brevity, the present application does not provide an exhaustive list of specific point values included in the range, and a range from 5 to 20 is further preferred.

Preferably, the pressure at the top of the rectification column is in a range from 0.1 kPa to 4 kPa, for example, it can be 0.2 kPa, 0.5 kPa, 0.8 kPa, 1 kPa, 1.2 kPa, 1.5 kPa, 1.8 kPa, 2 kPa, 2.2 kPa, 2.5 kPa, 2.8 kPa, 3 kPa, 3.2 kPa, 3.5 kPa or 3.8 kPa, as well as specific point values between the above point values. Due to the limited space and for the sake of brevity, the present application does not provide an exhaustive list of specific point values included in the range, and a range from 0.15 kPa to 2.5 kPa is further preferred.

Preferably, the reflux ratio at the top of the rectification column is in a range from 0.01 to 40, for example, it can be 0.05, 0.1, 0.5, 1, 3, 5, 8, 10, 12, 15, 18, 20, 22, 25, 28, 30, 32, 35 or 38, as well as specific point values between the above point values. Due to the limited space and for the sake of brevity, the present application does not provide an exhaustive list of specific point values included in the range, and a range from 0.1 to 20 is further preferred.

In a preferred technical solution of the present application, the method for preparing the isocyanate composition comprises the following steps:

(1) phosgenation process: mixing a diamine with a solvent to obtain an amine solution; introducing phosgene into the amine solution to carry out a reaction to obtain a reaction product; wherein the reaction comprising a cold reaction and a hot reaction carried out sequentially, and the temperature of the cold reaction is less than the temperature of the hot reaction;

(2) removal process: carrying out a removal treatment on the reaction product obtained in step (1) to obtain a crude product; wherein the removal treatment comprises a phosgene removal treatment and/or a solvent removal treatment;

(3a) separation process: separating the crude product obtained in step (2) to obtain a heavy component and an intermediate (light component);

(3b) heavy component recovery process: mixing the intermediate and heavy component obtained in step (3a) to obtain a mixture; wherein the mass percentage content of heavy component in the mixture is in a range from 1% to 10%; or, carrying out a secondary separation of the heavy component obtained in step (3a) to obtain a heavy component recovery material and a residual heavy component; mixing the heavy component recovery material with the intermediate to obtain a mixture; wherein the mass percentage content of heavy component in the mixture is in a range from 1% to 10%; and

(3c) refining process: refining the mixture obtained in step (3b) to obtain the isocyanate composition.

Exemplarily, a flow diagram of the method is shown in FIG. 1, comprising a phosgenation process 10, a removal process 20, a separation process 30, a heavy component recovery process 40, and a refining process 50. The phosgenation process may be implemented in an intermittent manner or in a continuous manner, and the continuous manner is carried out continuously by kettle type. The effective factor of the isocyanate composition is adjusted by appropriately adjusting the mixing ratio of the heavy component and the intermediate, the supply ratio of the phosgene, the reaction temperature, the reaction pressure, the average retention time, the reflux ratio of the rectification column, etc., and the control of the effective factor is mainly realized by adjusting the ratio of the heavy component and the intermediate.

Specifically, taking a NDI composition as an example, the method for preparing the NDI composition is as follows:

(1) phosgenation process: adopting a kettle reaction with three or four kettles continuous, continuously conveying amine solution containing NDA (naphthalene diamine) to the phosgenation reactor, and continuously introducing phosgene into the tops of a first kettle of cold reaction, a first kettle of hot reaction and a second kettle of hot reaction in the above ratio by inserting tubes; then, maintaining the interior of the first kettle of cold reaction at the temperature and reaction pressure of the cold reaction, while stirring and mixing the amine solution with phosgene; thus, NDA and phosgene (carbonyl chloride) undergoing a cold reaction to obtain a cold reaction photochemical liquid;

then, continuously conveying the cold reaction photochemical liquid to the top of the first kettle of hot reaction; that is, continuously supplying amine solution and phosgene to the first kettle of cold reaction, while continuously

taking out the photochemical liquid of cold reaction from the first kettle of cold reaction and transporting it to the first kettle of hot reaction;

next, maintaining the interior of the first kettle of hot reaction at the temperature and reaction pressure of the hot reaction, stirring and mixing the reaction substance and phosgene in the first kettle of hot reaction, and performing phosgenation reaction; similarly, carrying out phosgenation reaction in the second kettle of hot reaction while inputting reaction substances;

thus, continuously performing phosgenation process to obtain a reaction solution containing NDI;

(2) removal process: adopting a tower for phosgene removal and a tower for solvent removal, and continuously delivering the reaction solution to the middle part of the tower for phosgene removal; removing phosgene and hydrogen chloride from the reaction solution through the tower for phosgene removal; then, removing the solvent in the reaction solution through the tower for solvent removal to obtain a crude NDI product;

(3a) separation process: separating the crude NDI product by using a short path evaporator, and removing the heavy component to obtain an intermediate and a primary heavy component;

(3b) heavy component recovery process: recovering the primary heavy component by a short path evaporator to obtain heavy component recovery material and residual heavy component, which can be recovered once or circularly; the mixture obtained by mixing the heavy component recovery material and the intermediate entering the refining process; wherein the mass percentage content of the heavy component recovery material in the mixture is in a range from 1% to 10%;

(3c) refining step: continuously conveying the mixture to the column of a rectification column; then, under the aforementioned rectification conditions (temperature at the bottom of the column, temperature at the top of the column, pressure at the top of the column, reflux ratio at the bottom of the column, reflux ratio at the top of the column and residence time), distilling off the low-boiling substances from the intermediate, and extracting the NDI composition from the lower part of the column.

As a result, an NDI composition comprising an NDI, a CNI, and a substance corresponding to the effective factor can be continuously manufactured.

In a third aspect, embodiments of the present application provide a modified composition of an isocyanate, and the modified composition is obtained by modification of an isocyanate composition as described in the first aspect; the modified composition comprises any one or a combination of at least two of groups (a) to (i): (a) isocyanurate group, (b) uretdione group, (c) biuret group, (d) urethane group, (e) urea group, (f) iminooxadiazinedione group, (g) allophanate group, (h) uretonimine group and (i) carbodiimide group.

Those skilled in the art can modify the isocyanate composition as desired using known methods to obtain the modified composition; the modified composition as a polyisocyanate component, and a substance containing an active hydrogen group as a raw material for an isocyanate-based polymer such as a polyurethane resin are suitably utilized.

Specifically, a modified composition comprising the group (a) isocyanurate group is a trimer of isocyanate, which can be obtained, for example, by reacting an isocyanate composition in the presence of a known isocyanurate esterification catalyst to isocyanurate the isocyanate therein.

A modified composition comprising the group (b) uretdione group may be obtained by heating an isocyanate composition at a temperature in a range from 90°C to 200°C, or by reacting it in the presence of a known uretdione catalyst, and subjecting isocyanate to uretdione (for example, dimerization).

A modified composition comprising the group (c) biuret group may be obtained by reacting an isocyanate composition with, for example, water, a tertiary alcohol (for example, tert-butanol, etc.), a secondary amine (for example, dimethylamine, diethylamine, etc.), etc., and further reacting the composition in the presence of a known biuretization catalyst.

A modified composition comprising the group (d) urethane group can be obtained by reacting an isocyanate composition with a polyol (for example, trimethylolpropane, etc.).

A modified composition comprising the group (e) urea group may be obtained by reacting an isocyanate composition with water, polyamine (hereinafter referred to) and the like.

A modified composition comprising the group (f) iminooxadiazinedione group, i.e., asymmetric trimers of isocyanates, may be obtained by reacting an isocyanate composition in the presence of a known iminooxadiazinedione catalyst, subjecting the isocyanate to iminooxadiazinedione (for example, trimerisation).

A modified composition comprising the group (g) allophanate group may be obtained by reacting an isocyanate

composition with an alcohol and then further reacting it in the presence of a known allophanation catalyst.

A modified composition comprising the group (h) uretonimine group may be obtained by reacting an isocyanate composition in the presence of a known carbodiimidisation catalyst to form a carbodiimide group followed by addition of an isocyanate to the carbodiimide group.

A modified composition comprising the group (i) carbodiimide group may be obtained by reacting an isocyanate composition in the presence of a known carbodiimidisation catalyst.

It should be noted that the modified composition may contain at least one of the above groups (a)-(i), or at least two groups. Such a modified composition can be generated by appropriately combining the above reactions. Alternatively, the modified composition may be used alone or in combination of two or more.

In a fourth aspect, embodiments of the present application provide an isocyanate-based polymer, wherein the isocyanate-based polymer is formed by reacting an isocyanate substance with a substance containing an active hydrogen group; and the isocyanate substance comprises at least one of the isocyanate composition as described in the first aspect and the modified composition as described in the third aspect.

Preferably, the active hydrogen group comprises any one or a combination of at least two of hydroxyl, amino and mercapto.

Preferably, the substance containing an active hydrogen group comprises any one or a combination of at least two of a polyol, a polyamine and a polythiol.

The substance containing an active hydrogen group is a polyol and the isocyanate-based polymer is a polyurethane; the substance containing an active hydrogen group is a polyamine and the isocyanate-based polymer is a polyurea; and the substance containing an active hydrogen group is a polythiol and the isocyanate-based polymer is a polyurethane.

In a fifth aspect, embodiments of the present application provide a polyurethane elastomer, and the raw materials for preparing the polyurethane elastomer comprise an isocyanate substance and a polyol; the isocyanate substance comprises the isocyanate composition in the first aspect and/or the modified composition as described in the third aspect.

Preferably, the polyol is a high molecular weight polyol.

Preferably, the raw material further comprises a chain extender.

Preferably, the chain extender comprises a low molecular weight polyol and/or a low molecular weight polyamine.

In the present application, the polyurethane elastomer (PUR) may be a thermoplastic polyurethane elastomer (TPU), a thermosetting polyurethane elastomer (TSU), a rollable polyurethane elastomer and the like. The polyurethane elastomer comprises soft chain segments formed by the reaction of an isocyanate substance with a polyol (a high molecular weight polyol), and hard chain segments formed by the reaction of an isocyanate substance with a chain extender (a low molecular weight polyol and/or a low molecular weight polyamine). The polyurethane elastomer can be manufactured by the reaction of an isocyanate substance, a high molecular weight polyol (a substance containing an active hydrogen group), a low molecular weight polyol, and/or a low molecular weight polyamine (a substance containing an active hydrogen group). That is, the isocyanate substance (the isocyanate composition and/or the modified composition), the polyol (high molecular weight polyol), and the chain extender (low molecular weight polyol and/or low molecular weight polyamine) are the raw materials for the preparation of the polyurethane elastomer.

In the present application, the isocyanate substance comprises the isocyanate composition and/or the modified composition, the isocyanate composition has an effective factor ranging from 3.80 to 5.30, and the modified composition is obtained by modifying the isocyanate composition with an effective factor ranging from 3.80 to 5.30. The present application can effectively inhibit the discoloration of the polyurethane elastomer, improve the tensile strength and tear strength of the polyurethane elastomer through the design and control of the effective factor, so that the polyurethane elastomer exhibits excellent comprehensive performance in terms of discoloration resistance, weather resistance, stability, and mechanical properties.

Preferably, the polyol is a high molecular weight polyol, exemplarily including but not limited to: any one or a combination of at least two of a polyester polyol, a polycarbonate polyol, a polyether polyol, further preferably a polyester polyol.

Preferably, the polyester polyol comprises a polycaprolactone polyol and/or an adipic acid-based polyester polyol (a polyester polyol with adipic acid as a polyacid), further preferably an adipic acid-based polyester polyol.

Preferably, the polyether polyol comprises polytetramethylene ether glycol.

Preferably, the chain extender comprises a low molecular weight polyol and/or a low molecular weight polyamine, further preferably a low molecular weight polyol.

Preferably, the low molecular weight polyol comprises ethylene glycol and/or 1,4-butanediol, further preferably 1,4-butanediol.

In the present application, the polyurethane elastomer can be prepared by known methods such as the one shot method or the prepolymer method.

For the one shot method, for example, the isocyanate substance (the polyisocyanate composition, the isocyanate composition and/or the modified composition), the polyol (the high molecular weight polyol), the chain

extender (the low molecular weight polyol and/or the low molecular weight polyamine) are reacted in a single shot to obtain the polyurethane elastomer.

For the prepolymer method, for example, first, the isocyanate substance (the polyisocyanate composition, the isocyanate composition and/or the modified composition) is reacted with the polyol (the high molecular weight polyol) to synthesise a prepolymer having an isocyanate group at the end of the molecule; and then, the prepolymer is reacted with the chain extender (the low molecular weight polyol and/or the low molecular weight polyamine) to obtain the polyurethane elastomer.

It is to be noted that with respect to the method for preparing the polyurethane elastomer, polymerization methods known in the art may be used, such as bulk polymerization, solution polymerization and the like.

Further, for the preparation of the polyurethane elastomer, a known carbamate catalyst such as, an amine, an organometallic compound (for example, an organotin-based compound, preferably dibutyltin dichloride, tin octanoate, etc.) may be added to the raw material as needed.

Plasticizer, anti-caking agent, heat-resistant stabilizer, light-resistant stabilizer, ultraviolet absorber, NOx yellowing inhibitor, antioxidant, mold release agent, pigment, dye, lubricant, nucleating agent, filler, anti-hydrolysis agent and the like, can be combined with the polyurethane elastomers in appropriate proportions, as needed.

As a preferred technical solution of the present application, the polyurethane elastomer has excellent discoloration resistance and weather resistance, and excellent mechanical properties (tensile strength and tear strength).

In particular, the polyurethane elastomer has a color difference $\Delta b \leq 3.0$ after a wet heat aging test under xenon lamp irradiation for 240 h, for example $\Delta b$ can be 2.95, 2.9, 2.85, 2.8, 2.75, 2.7, 2.65, 2.6, 2.55, 2.5, 2.45, 2.4, 2.35, 2.3, 2.25, 2.2, 2.15, 2.1, 2.05, 2.0, 1.9, 1.8, 1.7, 1.6, 1.5, etc., further preferably < 3.0.

Preferably, the polyurethane elastomer prepared with the NDI composition has a tensile strength > 41 MPa, up to 41.3 MPa to 42.5 MPa, and a tear strength > 64 kN/m, up to 64.1 kN/m to 65.2 kN/m.

Preferably, the polyurethane elastomer prepared with the PPDI composition has a tensile strength > 44 MPa, up to 44.3 MPa to 45.7 MPa; a tear strength > 119 kN/m, up to 119.6 kN/m to 121.6 kN/m.

Preferably, the polyurethane elastomer prepared with the CHDI composition has a tensile strength > 35 MPa, up to 35.1 MPa to 36.1 MPa; and a tear strength $\geq$ 59 kN/m, 59.0 kN/m to 60.1 kN/m.

Compared with the prior art, the embodiments of the present application have the following beneficial effects.

In the isocyanate composition provided in the embodiments of the present application, through the design and control of effective factors, it has excellent reactivity and can be used for the preparation of high-performance polyurethane products. The isocyanate composition can effectively improve the stability of the polyurethane product, and in particular can significantly improve the discoloration resistance and weather resistance of the polyurethane elastomer, inhibit color increase and the yellowing under humid and hot conditions, make the color difference of the polyurethane elastomer $\Delta b$ is <3.0 after a wet heat aging test of the polyurethane elastomer under a xenon lamp irradiation of 240 h, and improve the tensile strength and tear strength of the polyurethane elastomer, so that the polyurethane elastomer has an excellent overall performance in terms of weather resistance and mechanical strength.

Other aspects can be understood after reading and understanding the accompanying drawings and the detailed description.

## DESCRIPTION OF THE DRAWINGS

The accompanying drawings are used to provide a further understanding of the technical solutions herein and form part of the specification and are used in conjunction with the examples of the present application to explain the technical solutions herein and do not constitute a limitation of the technical solutions herein.

FIG. 1 shows a flowchart of a method for preparing an isocyanate composition described in one specific embodiment of the present application;

wherein, 10-phosgenation process, 20-removal process, 30-separation process, 40-heavy component recovery process, 50-refining process.

## DETAILED DESCRIPTION OF THE INVENTION

The technical solution of the present application will be further illustrated below by way of specific embodiments. It should be clear to those skilled in the art that the described examples are merely only for the purpose of helping to understand the present application and should not be regarded as a specific limitation of the present application.

The components and properties in the present application are tested as follows:

1. Determination of the mass content (B-value) of chloroisocyanate in the isocyanate composition: GC-MS test

The analysis is carried out using gas chromatography-mass spectrometry under the following conditions. The

content herein is the normalized content.
Analytical instrument: Agilent 5977B GCMS
Column: DB-5 chromatographic column, with specifications of 30m×0.25mm×0.25μm.
Column temperature: keeping 50°C for 2min, heating to 80°C at a speed of 5mL/min, then heating to 280°C at a speed of 15mL/min and keeping it for 10min.
Separation ratio: no shunt
Inlet temperature: 280°C
Detection temperature: 300°C
Carrier gas: helium gas
Carrier gas flow: 1mL/min (constant flow)
Injection volume: 1μL
Detection method: SIM selected ion scanning mode (for NDI selected ion 202/168, for PPDI selected ion 152/118, for CHDI selected ion 158/124)

2. Determination of the mass content (A value) of chlorine in isocyanate compositions: XRF test

Instrument: Energy dispersive X-ray fluorescence spectroscopy (ED-XRF), Model: MERAK-LE II;
Method: Standard addition method
Principle and operation: chromatographically pure $CCl_4$ standard is the source of Cl, ethyl acetate is the diluent, the X-ray generated by the X-ray phototube excites Cl element in the sample and produces the characteristic X-ray fluorescence, the intensity of characteristic X-ray fluorescence is linearly related to the elemental concentration. A standard curve is plotted, and the extrapolated value is the content of Cl element in the sample.

3. Determination of the mass percentage content of isocyanate in the isocyanate composition: gas chromatography test

The analysis is carried out using gas chromatography under the following conditions. The content herein is the normalized content.
Analytical instrument: Agilent 7890B GC
Column: DB-5 chromatographic column, with specifications of 30m×0.25mm×0.25μm.
Column temperature: keeping 60°C for 1min, heating to 280°C at a speed of 10°C/min and keeping it for 5min.
Separation ratio: 30:1
Inlet temperature: 280°C
Detection temperature: 320°C
Carrier gas: nitrogen gas
Carrier gas flow: 1mL/min (constant flow)
Injection volume: 1μL
Detector: FID
In the following specific embodiments of this application, unless otherwise specified, "parts" and "%" are based on mass.

Example 1

An NDI composition and a method for preparing the same, wherein the NDI composition has an effective factor E of 5.30, and a flow diagram of the preparation method is shown in FIG. 1, specifically including the following steps: Phosgenation process: 800 parts by mass of chlorobenzene was put into a cold reaction kettle, the temperature in the cold reaction kettle was adjusted to 20°C, and the pressure (gauge pressure) was adjusted to 0.04 MPaG. 1,5-NDA (1,5-naphthalenediamine), a solution of 1,5-NDA (1,5-naphthalenediamine) in chlorobenzene with the concentration of 10.0 wt% was continuously charged into the cold reaction kettle at a rate of 500 mass parts/h, and phosgene was continuously introduced into the reactor at the rate of 626 mass parts/h, and the cold reaction was performed at a temperature of 20°C for 2.5 h to obtain a cold reaction photochemical liquid.
The photochemical liquid of cold reaction was taken out from the cold reaction kettle and transported to a hot reaction kettle, and phosgene was introduced into the hot reaction kettle at a rate of mass parts/h, and the temperature in the hot reaction kettle was maintained at 110°C, and the pressure (gauge pressure) was adjusted to 0.2 MPa G, and the hot reaction was carried out at 110°C for 4 h, so that 1,5-NDA reacted with phosgene to generate 1,5-NDI, and a reaction product comprising 1,5-NDI was prepared.
Removal process: the reaction product obtained from the phosgenation process was continuously conveyed to the tower for phosgene removal and the tower for solvent removal to perform phosgene removal treatment and solvent

removal treatment, respectively, whereby 130 mass parts of a crude product of NDI was prepared.

Separation process: the crude product obtained in the removal process was continuously conveyed to a short path evaporator, and 117.2 mass parts of intermediate and 12.8 mass parts of primary heavy component were obtained by removing heavy component.

Heavy component recovery process: the primary heavy component was continuously conveyed to the secondary short path evaporator, obtaining 2.39 mass parts of heavy component recovery material and 10.4 mass parts of residual heavy component. Next, an intermediate at a rate of 117.2 mass parts/h was mixed with the heavy component recovery material at a rate of 2.39 mass parts/h to obtain a mixture of 119.6 mass parts/h, i.e. the mass percentage of the heavy component recovery material in the mixture was 2%.

Refining process: the mixture was continuously conveyed into a rectification column, for which the rectification column was filled with a filler equivalent to a number of theoretical plates of 5. Then, in the rectification column, the light component was removed from the top of the column and the NDI composition was extracted from the column to obtain the target product.

The rectification conditions in the rectification column are shown below:

Temperature at the bottom of column: 130-140°C
Temperature at the top of column: 120-130°C
Pressure at the top of column: 0-50 PaA
Residence time: 2-4h
Reflux ratio at the top of column: 4
Thereby, the NDI composition was obtained, wherein the mass content of NDI was >99%, the mass content of chlorine (A value) was 7.6 ppm, the mass content of chloroisocyanate CNI (B value) was 15 ppm, and the effective factor E was 5.30.

Examples 2 to 5, Comparative Examples 1 to 2

An NDI composition and a method for preparing the same, the effective factor E of the NDI composition is shown in Table 1, respectively, and the process of the preparation method thereof is the same as that of Example 1, with the difference being only in some of the process parameters, which are shown in Table 1 (the process/parameters not shown in Table 1 are exactly the same as that of Example 1). In Table 1, "phosgene molar ratio" indicates the molar amount of the phosgene in the phosgenation process with 1,5-NDA as 1 mol, and "heavy component recovery material ratio" indicates the mass percentage of the heavy component (recovery material) in the mixture in the heavy component recovery process.

Table 1

| | | Example 1 | Example 2 | Example 3 | Example 4 | Example 5 | Comparative Example 1 | Comparative Example 2 |
|---|---|---|---|---|---|---|---|---|
| Phosgenation process | Phosgene inflow rate (mass parts/h) | 626 | 626 | 782 | 782 | 1220 | 626 | 1220 |
| | Phosgene molar ratio | 10 | 10 | 12.5 | 12.5 | 19.5 | 10 | 19.5 |
| | Temperature of cold reaction (°C) | 20 | 20 | 40 | 40 | 40 | 20 | 40 |
| | Temperature of hot reaction (°C) | 110 | 110 | 115 | 115 | 115 | 110 | 115 |
| | Reaction pressure (MPa G) | 0.2 | 0.2 | 0.2 | 0.2 | 0.2 | 0.2 | 0.2 |
| Heavy component recovery process | Mixture (mass parts/h) | 119.6 | 119.4 | 118.7 | 118.5 | 118.2 | 117.2 | 120.6 |
| | Heavy component recovery material (mass parts/h) | 2.39 | 5.37 | 7.72 | 10.07 | 11.82 | 0.58 | 14.472 |
| | Heavy component recovery material ratio (%) | 2 | 4.5 | 6.5 | 8.5 | 10 | 0.5 | 12 |
| Refining process | Yield (mass parts/h) | 114.8 | 115 | 114 | 113.7 | 113.4 | 113.2 | 115.4 |
| | Reflux ratio | 4 | 3 | 3 | 4 | 4 | 4 | 4 |
| NDI composition | 1,5-NDI (%) | > 99 | > 99 | > 99 | > 99 | > 99 | > 99 | > 99 |
| | Chlorine (ppm) | 7.6 | 47.8 | 147.5 | 242 | 376 | 5.6 | 398 |
| | CNI (ppm) | 15 | 125 | 474 | 765 | 1250 | 15 | 1250 |
| | Effective factor E | 5.30 | 4.59 | 4.19 | 3.96 | 3.80 | 5.52 | 3.77 |

Examples 6 to 10 and Comparative Examples 3 to 4

A PPDI composition and a method for preparing the same, the effective factor E of the PPDI composition is shown in Table 2, respectively, and the process of the preparation method thereof is the same as that of Example 1, with the difference being only in some of the process parameters, which are shown in Table 2 (the process/parameters not shown in Table 2 are exactly the same as that of Example 1). In Table 2, "phosgene molar ratio" indicates the molar amount of the phosgene in the phosgenation process with 1,4-diaminobenzene as 1 mol; and "heavy component recovery material ratio" indicates the mass percentage of the heavy component (recovery material) in the mixture in the heavy component recovery process.

Table 2

| | | Example 6 | Example 7 | Example 8 | Example 9 | Example 10 | Comparative Example 3 | Comparative Example 4 |
|---|---|---|---|---|---|---|---|---|
| Phosgenation process | Phosgene inflow rate (mass parts/h) | 915 | 915 | 1144 | 1144 | 1785 | 915 | 1785 |
| | Phosgene molar ratio | 10 | 10 | 12.5 | 12.5 | 19.5 | 10 | 19.5 |
| | Temperature of cold reaction (°C) | 20 | 20 | 40 | 40 | 40 | 20 | 40 |
| | Temperature of hot reaction (°C) | 110 | 110 | 115 | 115 | 115 | 110 | 115 |
| | Reaction pressure (MPa G) | 0.2 | 0.2 | 0.2 | 0.2 | 0.2 | 0.2 | 0.2 |
| Heavy component recovery process | Mixture (mass parts/h) | 133.3 | 133.1 | 132.3 | 132.1 | 131.7 | 130.6 | 134.4 |
| | Heavy component recovery material (mass parts/h) | 2.67 | 5.99 | 8.60 | 11.23 | 13.17 | 0.65 | 16.13 |
| | Heavy component recovery material ratio (%) | 2 | 4.5 | 6.5 | 8.5 | 10 | 0.5 | 12 |
| Refining process | Yield (mass parts/h) | 127.9 | 128.2 | 127.1 | 126.7 | 126.4 | 126.2 | 128.6 |
| | Reflux ratio | 4 | 3 | 3 | 4 | 4 | 4 | 4 |
| **PPDI** composition | 1,4-PPDI (%) | > 99 | > 99 | > 99 | > 99 | > 99 | > 99 | > 99 |
| | Chlorine (ppm) | 8 | 44 | 145 | 223 | 377.7 | 5.4 | 408 |
| | CPPI (ppm) | 13 | 127 | 345 | 634 | 950 | 13 | 950 |
| | Effective factor E | 5.30 | 4.83 | 4.19 | 4.12 | 3.80 | 5.62 | 3.72 |

Examples 11-15 and Comparative Examples 5-6

A CHDI composition and a method for preparing the same, the effective factor E of said PPDI composition is shown in Table 3, respectively, and the process of the preparation method thereof is the same as that of Example 1, with the difference being only in some of the process parameters, which are shown in Table 3 (the process/parameters not shown in Table 3 are exactly the same as that of Example 1). In Table 3, "phosgene molar ratio" indicates the molar amount of the phosgene in the phosgenation process with 1,4-diaminocyclohexane as 1 mol, and "heavy component recovery material ratio" indicates the mass percentage of the heavy component (recovery material) in the mixture in the heavy component recovery process.

Table 3

| | | Example 11 | Example 12 | Example 13 | Example 14 | Example 15 | Comparative Example 5 | Comparative Example 6 |
|---|---|---|---|---|---|---|---|---|
| Phosgenation process | Phosgene inflow rate (mass parts/h) | 867 | 867 | 1083 | 1083 | 1690 | 867 | 1690 |
| | Phosgene molar ratio | 10 | 10 | 12.5 | 12.5 | 19.5 | 10 | 19.5 |
| | Temperature of cold reaction (°C) | 20 | 20 | 40 | 40 | 40 | 20 | 40 |
| | Temperature of hot reaction (°C) | 110 | 110 | 115 | 115 | 115 | 110 | 115 |
| | Reaction pressure (MPa G) | 0.2 | 0.2 | 0.2 | 0.2 | 0.2 | 0.2 | 0.2 |
| Heavy component recovery process | Mixture (mass parts/h) | 131.0 | 130.7 | 130.0 | 129.8 | 129.4 | 128.3 | 132.1 |
| | Heavy component recovery material (mass parts/h) | 2.62 | 5.88 | 8.45 | 11.03 | 12.94 | 0.64 | 15.85 |
| | Heavy component recovery material ratio (%) | 2 | 4.5 | 6.5 | 8.5 | 10 | 0.5 | 12 |
| Refining process | Yield (mass parts/h) | 125.7 | 125.9 | 124.8 | 124.5 | 124.2 | 124.0 | 126.4 |
| | Reflux ratio | 4 | 3 | 3 | 4 | 4 | 4 | 4 |
| CHDI composition | 1,4-CHDI (%) | > 99 | > 99 | > 99 | > 99 | > 99 | > 99 | > 99 |
| | Chlorine (ppm) | 8.4 | 42.8 | 141.5 | 242 | 365 | 5.5 | 408 |
| | CCHI (ppm) | 16 | 134 | 384 | 654 | 990 | 16 | 990 |
| | Effective factor E | 5.30 | 4.83 | 4.21 | 3.98 | 3.80 | 5.67 | 3.70 |

Examples 16 to 18 and Comparative Examples 7 to 9

NDI was prepared by the method in Example 1 of prior art CN110256296A as Comparative Example 7, which was a thermal cracking method, and the product contained no chlorine, which means that it did not contain an effective factor; the heavy component recovery material in Example 1 was added to the product at a ratio of 4% (i.e., the mass percentage of heavy component in the mixture obtained was 4%), and Example 16 was obtained.

Similarly, adopting the method in Example 1 of CN110256296A, replacing the raw material NDA with PPDA, and other conditions being the same, PPDI was prepared as Comparative Example 8; and the heavy component recovery material in Example 6 was added to the product at a ratio of 4% (i.e., the mass percentage of heavy component in the mixture obtained was 4%), and Example 17 was obtained.

Adopting the method in Example 1 of CN110256296A, replacing the raw material NDA with CHDA, and other conditions being the same, CHDI was prepared as Comparative Example 9; the heavy component recovery material in Example 11 was added to the product at a ratio of 4% (i.e., the mass percentage content of heavy component in the mixture obtained was 4%), and Example 18 was obtained.

Application Example

A polyurethane elastomer, specifically a thermoplastic polyurethane elastomer (TPU), the raw material for the preparation of which comprises an isocyanate substance (a polyisocyanate composition), a high molecular weight polyol, and a chain extender (a low molecular weight polyol); the isocyanate substance is the isocyanate composition provided in Examples 1 to 15, and Comparative Examples 1 to 6, respectively, and the high molecular weight polyol is an adipic acid-based polyester polyol (TAKELAC U-2024 manufactured by Mitsui Chemical Company, number average molecular weight 2000), and the chain extender is 1,4-butanediol (Inokai reagent); furthermore, the raw materials further include a catalyst (tin octanoate) and a heat-resistant stabilizer (purchased from Ciba Specialty Chemicals, IRGANOX 245).

The polyurethane elastomer was prepared as follows:

(1) to a four-necked flask equipped with a stirrer, a thermometer, a reflux tube and a nitrogen supply line, 221 parts by mass of an NDI composition (168 parts by mass for the PPDI composition and 175 parts by mass for the CHDI composition) and 531.2 parts by mass of an adipic acid-based polyester polyol were charged, and the reaction was carried out under a nitrogen atmosphere at 80°C until the content of the NCO group was 9.1 wt.%, obtaining a prepolymer having an isocyanate group at the end of the molecule.
(2) 3.9 parts by mass of a heat-resistant stabilizer and 0.07 part by mass of a solution obtained by diluting the catalyst tin octoate to 4 wt.% by using diisononyl adipate (West Asia reagent) were added to the prepolymer obtained in the step (1), stirred and mixed by using a mechanical stirrer (IKA, RW20, Germany) at a rotation speed of 600 rpm for about 1 min, and then 131.9 parts by mass of 1,4- butanediol, which had been adjusted to 80°C in advance, was added into the system, and was fully stirred for about 2 min until it was evenly mixed to obtain a mixed solution.
(3) the mixed solution obtained in step (2) was flown into a stainless steel dish whose temperature had been adjusted to 150°C in advance, and reacted at 150°C for 1 h, and then reacted at 100°C for 23 h to obtain the elastomer.
(4) the elastomer obtained in step (3) was removed from the dish and cured under constant temperature and humidity conditions of 23°C and a relative humidity of 55% for 7 days to obtain the polyurethane elastomer.

Performance evaluation of the polyurethane elastomer

The polyurethane elastomer to be tested (raw material, the mixture obtained in step (2) of the example of application) was subjected to injection molding using an injection molding machine (model: NEX-140, Taifu Machinery) at a screw rotation speed of 100 rpm, a barrel temperature of 150-235°C, and was implemented at a mold temperature of 20°C, an injection time of 10 s, an injection speed of 60 mm/s, and a cooling time of 45 s at a mold temperature of 20°C, an injection speed of 60 mm/s, and a cooling time of 45 s to obtain a sheet.

The obtained sheet (with a thickness of 2 mm) was maintained for 7 days at a constant temperature and humidity of 23°C and a relative humidity of 55% to obtain a polyurethane elastomer sheet for testing, and the following performance tests were specifically carried out:

(1) firstly, the B value (b1, initial value) of polyurethane elastomer sheet was measured by a color colorimeter; then the xenon lamp irradiation test was carried out, and the polyurethane elastomer sheet was placed in a super xenon lamp climate test box (Weibang Instrument) for 240 h under the conditions of temperature of 89°C, relative humidity of 50% and xenon lamp irradiation of 100 W/$m^2$ (irradiation wavelength of 300-400 nm), and then the sheet was taken out, and the B value of the sheet was tested by the same method as above (b2). The color difference $\Delta b$ of polyurethane elastomer after 240 h of wet heat aging test under xenon lamp irradiation was calculated, $\Delta b = | b2-b1 |$;

(2) Tensile strength: the tensile strength of the elastomer was tested according to the method in GB/T 528-2009;
(3) Tear strength: the tear strength of the elastomer was tested according to the method in GB/T 529-2008;

The foregoing test results are shown in Table 4, Table 5 and Table 6.

Table 4

| | | Ex. 1 | Ex. 2 | Ex. 3 | Ex. 4 | Ex. 5 | Ex. 16 | Comp. Ex. 1 | Comp. Ex. 2 | Comp. Ex. 7 |
|---|---|---|---|---|---|---|---|---|---|---|
| **NDI** compositi on | 1,5-NDI (%)) | > 99 | > 99 | > 99 | > 99 | > 99 | > 99 | > 99 | > 99 | > 99 |
| | Chlorine (ppm) | 7.6 | 47.8 | 147.5 | 242 | 376 | 9.6 | 5.6 | 408 | - |
| | CNI (ppm) | 15 | 125 | 474 | 765 | 1250 | 18 | 15 | 1250 | - |
| | Effective factor E | 5.30 | 4.59 | 4.19 | 3.96 | 3.80 | 5.19 | 5.52 | 3.77 | - |
| **TPU** | b1 | 0.51 | 0.52 | 0.53 | 0.55 | 0.56 | 0.52 | 0.52 | 0.65 | 0.51 |
| | b2 | 3.1 | 3.21 | 3.27 | 3.3 | 3.51 | 3.24 | 4.85 | 5.25 | 4.84 |
| | Δb | 2.59 | 2.69 | 2.74 | 2.75 | 2.95 | 2.72 | 4.33 | 4.60 | 4.32 |
| | Tensile strength (MPa) | 42.5 | 42.3 | 42.0 | 41.6 | 41.3 | 41.9 | 38.1 | 32.3 | 32.2 |
| | Tear strength (kN/m) | 65.1 | 65.2 | 64.6 | 64.4 | 64.1 | 65.0 | 55.1 | 54.3 | 53.2 |

Table 5

| | | Ex. 6 | Ex. 7 | Ex. 8 | Ex. 9 | Ex. 10 | Ex. 17 | Comp. Ex. 3 | Comp. Ex. 4 | Comp. Ex. 8 |
|---|---|---|---|---|---|---|---|---|---|---|
| **PPDI** comp ositio n | 1,4-**PPDI** (%) | > 99 | > 99 | > 99 | > 99 | > 99 | > 99 | > 99 | > 99 | > 99 |
| | Chlorine (ppm) | 8 | 44 | 145 | 223 | 377.7 | 10 | 5.4 | 408 | - |
| | CPPI (ppm) | 13 | 127 | 345 | 634 | 950 | 15 | 13 | 950 | - |
| | Effective factor E | 5.30 | 4.83 | 4.19 | 4.12 | 3.80 | 5.18 | 5.62 | 3.72 | - |
| **TPU** | b1 | 0.52 | 0.53 | 0.54 | 0.56 | 0.57 | 0.53 | 0.53 | 0.62 | 0.52 |
| | b2 | 3.12 | 3.22 | 3.22 | 3.32 | 3.52 | 3.21 | 4.87 | 5.18 | 4.61 |
| | Δb | 2.60 | 2.69 | 2.68 | 2.76 | 2.95 | 2.68 | 4.35 | 4.56 | 4.09 |
| | Tensile strength (MPa) | 45.7 | 45.6 | 45.3 | 44.6 | 44.3 | 45.0 | 41.0 | 34.7 | 34.6 |
| | Tear strength (kN/m) | 121.5 | 121.6 | 120.5 | 120.1 | 119.6 | 121.4 | 102.9 | 101.4 | 99.3 |

Table 5

| | | Ex. 11 | Ex. 12 | Ex. 13 | Ex. 14 | Ex. 15 | Ex. 18 | Comp. Ex. 5 | Comp. Ex. 6 | Comp. Ex. 9 |
|---|---|---|---|---|---|---|---|---|---|---|
| **CHDI** composition | 1,4-CHDI (%) | > 99 | > 99 | > 99 | > 99 | > 99 | > 99 | > 99 | > 99 | > 99 |
| | Chlorine (ppm) | 8.4 | 42.8 | 141.5 | 242 | 365 | 12 | 5.5 | 408 | -- |
| | CCHI (ppm) | 16 | 134 | 384 | 654 | 990 | 21 | 16 | 990 | -- |
| | Effective factor E | 5.30 | 4.83 | 4.21 | 3.98 | 3.80 | 5.13 | 5.67 | 3.70 | -- |
| **TPU** | b1 | 0.50 | 0.51 | 0.52 | 0.54 | 0.55 | 0.51 | 0.52 | 0.64 | 0.52 |
| | b2 | 3.04 | 3.14 | 3.14 | 3.23 | 3.43 | 3.11 | 4.76 | 5.18 | 4.63 |
| | Δb | 2.53 | 2.62 | 2.61 | 2.69 | 2.88 | 2.60 | 4.24 | 4.54 | 4.11 |
| | Tensile strength (MPa) | 36.1 | 36.0 | 35.6 | 35.3 | 35.1 | 35.5 | 32.3 | 27.3 | 27.4 |
| | Tear strength (kN/m) | 59.9 | 60.1 | 59.5 | 59.3 | 59.0 | 59.8 | 50.6 | 50.1 | 48.9 |

Combined with the above performance test data, it can be seen that the present application makes the obtained polyurethane elastomer based on the isocyanate composition have excellent stability and weather resistance by controlling the effective factor of the isocyanate composition being in a range from 3.80 to 5.30, the color difference Δb after wet heat aging treatment under xenon lamp irradiation for 240 h is less than 3.0, as low as 2.53 to 2.95, which inhibits color increase and yellowing under humid and hot conditions and improves the tensile strength and tear strength of the polyurethane elastomer. The tensile strength of polyurethane elastomer prepared from NDI composition is in range from 41.3 MPa to 42.5 MPa, and the tear strength is in range from 64.1 kN/m to 65.2 kN/m; the tensile strength of polyurethane elastomer prepared from PPDI composition is in range from 44.3 MPa to 45.7 MPa, and the tear strength is in range from 119.6 kN/m to 121.6 kN/m; the tensile strength of the polyurethane elastomer prepared from the CHDI composition is in range from 35.1 MPa to 36.1 MPa, and the tear strength is in range from 59.0 kN/m to 60.1 kN/m, so that the polyurethane elastomer maintains excellent comprehensive product properties such as appearance, weather resistance and mechanical properties. It can be seen that the isocyanate composition provided in the present application has better application prospects in polyurethane elastomers.

The applicant declares that the present application illustrates the isocyanate composition, the modified composition and the polyurethane elastomer of the present application by means of the above examples, but the present application is not limited to the above process steps, i.e. it does not imply that the present application must be relied on the above process steps in order to be implemented. It should be clear to those skilled in the art that any improvement of the present application, equivalent substitution of raw materials selected in the present application, addition of auxiliary components, selection of specific methods, etc., fall within the scope of protection and disclosure of the present application.

## Claims

**1.** An isocyanate composition, wherein an effective factor of the isocyanate composition is in a range from 3.80 to 5.30;

the calculation formula of the effective factor is shown in Formula I:

$$E = -\lg\left(A - \frac{B \times M_{Cl}}{M_B}\right)$$ Formula I;

wherein E is the effective factor;
A is the mass content of chlorine in the isocyanate composition;
B is the mass content of chloroisocyanate in the isocyanate composition;
$M_{Cl}$ is the relative atomic mass of chlorine; and
$M_B$ is the relative molecular mass of the chloroisocyanate.

**2.** The isocyanate composition according to claim 1, wherein the isocyanate is diisocyanate.

**3.** The isocyanate composition according to claim 2, wherein the isocyanate composition comprises any one or a combination of at least two of naphthalene diisocyanate, phenylene diisocyanate, cyclohexane diisocyanate, toluene diisocyanate and diphenylmethane diisocyanate.

**4.** The isocyanate composition according to any one of claims 1 to 3, wherein the mass percentage of isocyanate in the isocyanate composition is ≥97%.

**5.** The isocyanate composition according to any one of claims 1 to 4, wherein the chloroisocyanate is a compound obtained by replacing one NCO group in isocyanate with chlorine;
preferably, the chloroisocyanate comprises any one or a combination of at least two of

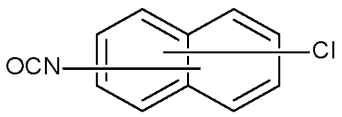 , 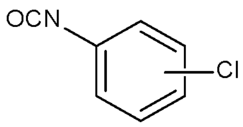 , 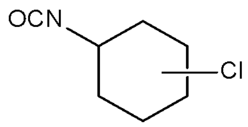 ,

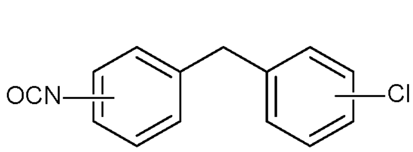 and 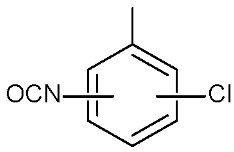 .

**6.** The isocyanate composition according to any one of claims 1 to 5, wherein a substance corresponding to the effective factor comprises any one or a combination of at least two of the following compounds:

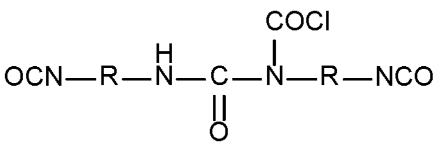 , 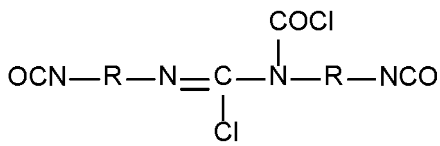 ,

and

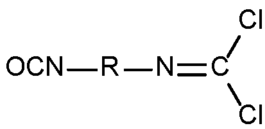 ,

wherein R is a divalent group obtained by removing NCO groups in isocyanate;
preferably, R is selected from any one or a combination of at least two of

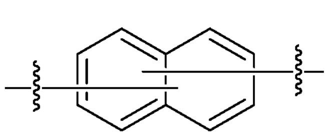 , 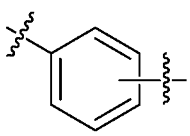 , 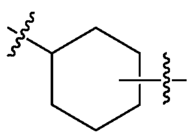 , 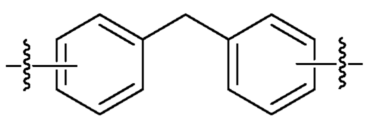

and

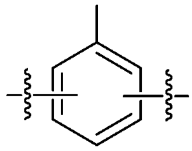

;

wherein a wavy line represents the connection sites of groups.

**7.** The isocyanate composition according to any one of claims 1 to 6, wherein the A is obtained by X-ray fluorescence spectrometry;
preferably, the B is obtained by chromatography-mass spectrometry, and further preferably by gas chromatography-mass spectrometry.

**8.** A method for preparing the isocyanate composition according to any one of claims 1 to 7, comprising: carrying out a reaction between an amine compound with phosgene to obtain the isocyanate composition.

**9.** The method according to claim 8, comprising:

(1) carrying out a reaction between the amine compound with phosgene to obtain a reaction product;
(2) carrying out a removal treatment on the reaction product obtained in step (1) to obtain a crude product; wherein the removal treatment comprises a phosgene removal treatment and/or a solvent removal treatment; and
(3) separating and refining the crude product obtained in step (2) in turn to obtain the isocyanate composition.

**10.** The method according to claim 8, wherein a heavy component and an intermediate are obtained by the separating in step (3); and a mixture of the intermediate and the heavy component is refined to obtain the isocyanate composition; and the mass percentage of the heavy component in the mixture is in a range from 1% to 10%.

**11.** The method according to claim 10, wherein the method for refining is rectification.

**12.** A modified composition of isocyanate, wherein the modified composition is obtained by modifying the isocyanate composition according to any one of claims 1 to 7;
and the modified composition comprises any one or a combination of at least two of groups (a) to (i): (a) isocyanurate group, (b) uretdione group, (c) biuret group, (d) urethane group, (e) urea group, (f) iminooxadiazinedione group, (g) allophanate group, (h) uretonimine group and (i) carbodiimide group.

**13.** An isocyanate-based polymer, wherein the isocyanate-based polymer is formed by reacting an isocyanate substance with a substance containing an active hydrogen group; and the isocyanate substance comprises at least one of the isocyanate composition according to any one of claims 1 to 7 and the modified composition according to claim 12.

**14.** A polyurethane elastomer, wherein the raw materials for preparing the polyurethane elastomer comprise an isocyanate substance and a polyol; the isocyanate substance comprises the isocyanate composition according to any one of claims 1 to 7 and/or the modified composition according to claim 12.

**15.** The polyurethane elastomer according to claim 14, wherein the raw material further comprises a chain extender; preferably, the chain extender comprises a low molecular weight polyol and/or a low molecular weight polyamine.

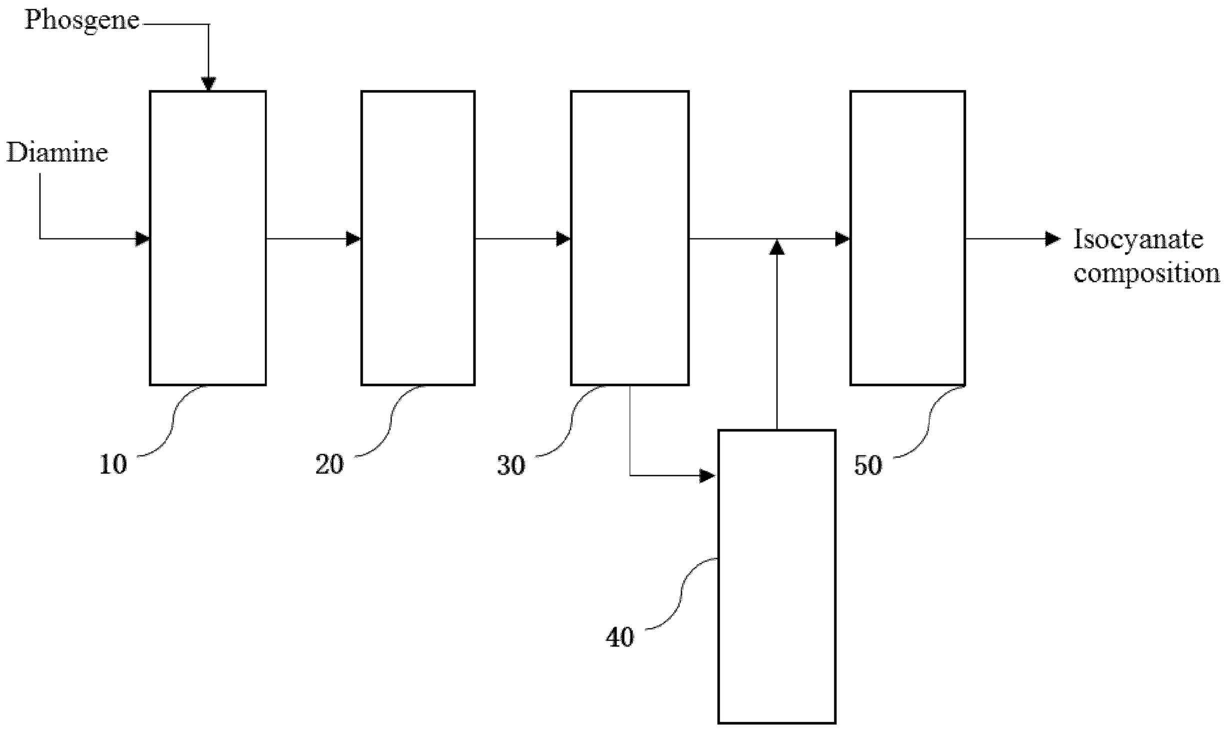


FIG. 1

## INTERNATIONAL SEARCH REPORT

International application No.
**PCT/CN2022/132424**

**A. CLASSIFICATION OF SUBJECT MATTER**

C08G18/71(2006.01)i; C08G18/75(2006.01)i; C08G18/76(2006.01)i; C08G18/77(2006.01)i; C08G18/78(2006.01)i; C08G18/79(2006.01)i; C08G18/66(2006.01)i; C08G18/42(2006.01)i; C08G18/32(2006.01)i; C07C265/14(2006.01)i

According to International Patent Classification (IPC) or to both national classification and IPC

**B. FIELDS SEARCHED**

Minimum documentation searched (classification system followed by classification symbols)

IPC:C08G C07C

Documentation searched other than minimum documentation to the extent that such documents are included in the fields searched

Electronic data base consulted during the international search (name of data base and, where practicable, search terms used)

CNABS; VEN; CNTXT; WPABSC; ENTXTC; WPABS; CJFD; CNKI; EPTXT; ISI web of science: 万华, 朱付林, 尚永华, 李建峰, 何伟, 吴谦, 王勤隆, 姜腾飞, 李强, 贾峥瑞, 黎源, 异氰酸酯, 氯, 光气, 碳酰氯, 二胺, 黄变, 变黄, 变色, 耐黄, 色号, 拉伸, 撕裂, X射线, chlorin+, +isocyanate?, NDI, CHDI, PPDI, phosgene, +diamine+

**C. DOCUMENTS CONSIDERED TO BE RELEVANT**

| Category* | Citation of document, with indication, where appropriate, of the relevant passages | Relevant to claim No. |
|---|---|---|
| A | CN 109153637 A (MITSUI CHEMICALS INC.) 04 January 2019 (2019-01-04) description, paragraphs 12-15, 53-56, and 65-88 | 1-15 |
| A | CN 104250363 A (ASAHI KASEI CHEMICALS CORP.) 31 December 2014 (2014-12-31) entire document | 1-15 |
| A | EP 4089071 A1 (MITSUI CHEMICALS INC.) 16 November 2022 (2022-11-16) entire document | 1-15 |
| A | CN 114920668 A (WANHUA CHEMICAL GROUP CO., LTD. et al.) 19 August 2022 (2022-08-19) entire document | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 July 2023** | **10 August 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2022/132424**

| Patent document cited in search report | Publication date (day/month/year) | Patent family member(s) | Publication date (day/month/year) |
|---|---|---|---|
| CN 109153637 A | 04 January 2019 | KR 20190129819 A | 20 November 2019 |
| | | US 2019292304 A1 | 26 September 2019 |
| | | US 10640605 B2 | 05 May 2020 |
| | | EP 3825301 A1 | 26 May 2021 |
| | | JP 2018177811 A | 15 November 2018 |
| | | JP 7103879 B2 | 20 July 2022 |
| | | BR 112018074934 A2 | 12 March 2019 |
| | | BR 112018074934 B1 | 28 April 2020 |
| | | EP 3470393 A1 | 17 April 2019 |
| | | EP 3470393 A4 | 11 March 2020 |
| | | EP 3470393 B1 | 03 March 2021 |
| | | WO 2018190290 A1 | 18 October 2018 |
| | | TW 201841880 A | 01 December 2018 |
| | | KR 20180127517 A | 28 November 2018 |
| | | KR 101970115 B1 | 17 April 2019 |
| | | JP 6373536 B1 | 15 August 2018 |
| | | VN 61467 A | 25 January 2019 |
| | | TW 655178 B1 | 01 April 2019 |
| | | IN 201817047391 A | 11 October 2019 |
| | | US 2020190249 A1 | 18 June 2020 |
| | | IN 338588 B | 19 June 2020 |
| | | VN 10024593 B | 27 July 2020 |
| | | CN 109153637 B | 08 December 2020 |
| | | CN 112409566 A | 26 February 2021 |
| | | CN 112409569 A | 26 February 2021 |
| | | CN 112409570 A | 26 February 2021 |
| | | CN 112480363 A | 12 March 2021 |
| | | CN 112480364 A | 12 March 2021 |
| | | EP 3825301 A1 | 26 May 2021 |
| | | US 11254783 B2 | 22 February 2022 |
| | | KR 20220025083 A | 03 March 2022 |
| | | KR 102491064 B1 | 19 January 2023 |
| CN 104250363 A | 31 December 2014 | JP 2015028163 A | 12 February 2015 |
| | | JP 6329017 B2 | 23 May 2018 |
| | | CN 104250363 B | 11 January 2017 |
| EP 4089071 A1 | 16 November 2022 | KR 20220110738 A | 09 August 2022 |
| | | CN 114787230 A | 22 July 2022 |
| | | WO 2022162968 A1 | 04 August 2022 |
| | | IN 202217030486 A | 16 September 2022 |
| | | JP 7289990 B2 | 12 June 2023 |
| | | EP 4089071 A4 | 21 June 2023 |
| CN 114920668 A | 19 August 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 113354788 A
- CN 104017166 A
- CN 104817683 A
- CN 110256296 A